# EUROPEAN PATENT SPECIFICATION

(11) **EP 2 986 640 B1**
(45) Date of publication and mention of the grant of the patent: **01.07.2020**
(21) Application number: 14785612.4
(22) Date of filing: 18.04.2014
(51) Int. Cl.: C07K 16/24, A61K 39/00, A61P 19/02, A61P 29/00, A61P 35/00

(54) **HUMANIZED ANTIBODY AGAINST INTERLEUKIN-20 AND TREATMENT FOR INFLAMMATORY DISEASES**
HUMANISIERTER ANTIKÖRPER GEGEN INTERLEUKIN-20 UND BEHANDLUNG FÜR ENTZÜNDLICHE ERKRANKUNGEN
ANTICORPS HUMANISÉS CONTRE L'INTERLEUKINE 20 ET TRAITEMENT POUR MALADIES INFLAMMATOIRES

(30) Priority: 18.04.2013 US 201313865671
(43) Date of publication of application: 24.02.2016
(73) Proprietor: Development Center for Biotechnology, New Taipei City 221 (TW); DCB-USA LLC, Wilmington, Delaware 19801 (US)
(72) Inventor: WU, Chia-Cheng, New Taipei City 221 (TW); HUANG, Chao-Yang, New Taipei City 221 (TW); LIN, Yu-Ying, Yangmei, Taoyuan (TW); CHEN, Yu-Jung, New Taipei City 221 (TW); LAI, Jiann-Shiun, New Taipei City 221 (TW)
(74) Representative: Osha Liang
(86) International application number: PCT/US2014/034579
(87) International publication number: WO 2014/172591

(56) References cited:
- WO-A1-2012/110360
- WO-A1-2013/177157
- WO-A2-2011/160119
- US-A- 5 874 299
- US-A1- 2006 193 850
- US-A1- 2012 308 575
- US-B1- 6 719 971
- US-B2- 7 786 274
- CHEUNG N K ET AL: "Humanizing murine IgG3 anti-GD2 antibody m3F8 substantially improves antibody-dependent cell-mediated cytotoxicity while retaining targeting in vivo", ONCOIMMUNOLOGY, LANDES BIOSCIENCE, US, vol. 1, no. 4, 1 July 2012 (2012-07-01), pages 477-486, XP002714906, ISSN: 2162-4011, DOI: 10.4161/ONCI.19864

### SEQUENCE LISTING

<110> Development Center for Biotechnology DCB-USA, LLC
<120> HUMANIZED ANTIBODY AGAINST INTERLEUKIN-20 AND TREATMENT FOR INFLAMMATORY DISEASES
<130> 17787/010WO1
<150> US 13/865,671
   <151> 2013-04-18
<160> 24
<170> PatentIn version 3.5
<210> 1
   <211> 121
   <212> PRT
   <213> Mus musculus
<400> 1
   Glu Leu Lys Leu Glu Glu Ser Gly Gly Gly Leu Val Gln Pro Gly Gly
   1               5                   10                  15
   Ser Met Lys Leu Ser Cys Ala Ala Ser Gly Phe Thr Phe Ser Asp Ala
               20                  25                  30
   Trp Met Asp Trp Val Arg Gln Ser Pro Glu Lys Gly Leu Glu Trp Ile
           35                  40                  45
   Ala Glu Ile Arg Ser Lys Ala Asn Asn Tyr Ala Thr Tyr Phe Ala Glu
       50                  55                  60
   Ser Val Lys Gly Arg Phe Thr Ile Ser Arg Asp Asp Ser Lys Ser Gly
   65                  70                  75                  80
   Val Tyr Leu Gln Met Asn Asn Leu Arg Ala Glu Asp Thr Gly Ile Tyr
                   85                  90                  95
   Phe Cys Thr Lys Leu Ser Leu Arg Tyr Trp Phe Phe Asp Val Trp Gly
               100                 105                 110
   Ala Gly Thr Thr Val Thr Val Ser Ser
           115                 120
<210> 2
   <211> 112
   <212> PRT
   <213> Homo sapiens
<400> 2
   Glu Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp His
               20                  25                  30
   Tyr Met Asp Trp Val Arg Gln Ala Pro Gly Lys Gly Leu Glu Trp Val
           35                  40                  45
   Gly Arg Thr Arg Asn Lys Ala Thr Ser Tyr Thr Thr Glu Tyr Ala Ala
       50                  55                  60
   Ser Val Lys Gly Arg Phe Thr Ile Ser Arg Asp Asp Ser Lys Asn Ser
   65                  70                  75                  80
   Leu Tyr Leu Gln Met Asn Ser Leu Lys Thr Glu Asp Thr Gly Val Tyr
                   85                  90                  95
   Tyr Cys Thr Lys Arg Trp Gly Gln Gly Thr Leu Val Thr Val Ser Ser
               100                 105                 110
<210> 3
   <211> 121
   <212> PRT
   <213> Artificial Sequence
<220>
   <223> Synthetic
<400> 3
   Glu Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ala
               20                  25                  30
   Trp Met Asp Trp Val Arg Gln Ala Pro Gly Lys Gly Leu Glu Trp Val
           35                  40                  45
   Gly Glu Ile Arg Ser Lys Ala Asn Asn Tyr Ala Thr Tyr Phe Ala Glu
       50                  55                  60
   Ser Val Lys Gly Arg Phe Thr Ile Ser Arg Asp Asp Ser Lys Asn Ser
   65                  70                  75                  80
   Leu Tyr Leu Gln Met Asn Ser Leu Lys Thr Glu Asp Thr Gly Val Tyr
                   85                  90                  95
   Tyr Cys Thr Lys Leu Ser Leu Arg Tyr Trp Phe Phe Asp Val Trp Gly
                   100                 105                 110
   Gln Gly Thr Leu Val Thr Val Ser Ser
           115                 120
<210> 4
   <211> 121
   <212> PRT
   <213> Artificial Sequence
<220>
   <223> Synthetic
<400> 4
   Glu Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ala
               20                  25                  30
   Trp Met Asp Trp Val Arg Gln Ala Pro Gly Lys Gly Leu Glu Trp Val
           35                  40                  45
   Gly Glu Ile Arg Ser Lys Ala Asn Asn Tyr Ala Thr Tyr Phe Ala Glu
       50                  55                  60
   Ser Val Lys Gly Arg Phe Thr Ile Ser Arg Asp Asp Ser Lys Asn Ser
   65                  70                  75                  80
   Leu Tyr Leu Gln Met Asn Ser Leu Lys Thr Glu Asp Thr Gly Val Tyr
                   85                  90                  95
   Tyr Cys Thr Lys Leu Ser Leu Arg Tyr Trp Phe Phe Asp Val Trp Gly
               100                 105                 110
   Gln Gly Thr Leu Val Thr Val Ser Ser
           115                 120
<210> 5
   <211> 113
   <212> PRT
   <213> Mus musculus
<400> 5
   Asp Phe Val Met Thr Gln Thr Pro Leu Thr Leu Ser Val Thr Ile Gly
   1               5                   10                  15
   Gln Pro Ala Ser Ile Ser Cys Lys Ser Ser Gln Ser Leu Leu Asp Ser
               20                  25                  30
   Asp Gly Lys Thr Tyr Leu Asn Trp Leu Leu Gln Arg Pro Gly Gln Ser
   35 40 45
   Pro Lys His Leu Ile Tyr Leu Val Ser Lys Leu Asp Ser Gly Val Pro
   50 55 60
   Asp Arg Phe Thr Gly Ser Gly Ser Gly Thr Asp Phe Thr Leu Arg Ile
   65 70 75 80
   Ser Arg Val Glu Ala Glu Asp Leu Gly Val Tyr Tyr Cys Trp Gln Ser
   85 90 95
   Thr His Phe Pro Trp Thr Phe Gly Gly Gly Thr Lys Leu Glu Ile Lys
   100 105 110
   Arg
<210> 6
   <211> 111
   <212> PRT
   <213> Homo sapiens
<400> 6
   Asp Ile Gln Met Thr Gln Ser Pro Leu Ser Leu Ser Val Thr Pro Gly
   1 5 10 15
   Gln Pro Ser Ile Ser Cys Lys Ser Ser Gln Leu Leu His Ser Asp Gly
   20 25 30
   Lys Thr Tyr Leu Tyr Trp Tyr Gln Gln Lys Pro Gly Gln Ser Pro Gln
   35 40 45
   Leu Leu Ile Tyr Glu Val Ser Asn Arg Phe Ser Gly Val Pro Asp Arg
   50 55 60
   Phe Ser Gly Ser Gly Ser Gly Thr Asp Phe Thr Leu Lys Ile Ser Arg
   65 70 75 80
   Val Glu Ala Glu Asp Val Gly Val Tyr Tyr Cys Met Gln Gly Ile Gln
   85 90 95
   Leu Pro Trp Thr Phe Gly Gln Gly Thr Lys Val Glu Ile Lys Arg
   100 105 110
<210> 7
   <211> 111
   <212> PRT
   <213> Artificial Sequence
<220>
   <223> Synthetic
<400> 7
   Asp Ile Gln Met Thr Gln Ser Pro Leu Ser Leu Ser Val Thr Pro Gly
   1               5                   10                  15
   Gln Pro Ser Ile Ser Cys Lys Ser Ser Gln Leu Leu Asp Ser Asp Gly
               20                  25                  30
   Lys Thr Tyr Leu Asn Trp Tyr Gln Gln Lys Pro Gly Gln Ser Pro Gln
           35                  40                  45
   Leu Leu Ile Tyr Leu Val Ser Lys Leu Asp Ser Gly Val Pro Asp Arg
       50                  55                  60
   Phe Ser Gly Ser Gly Ser Gly Thr Asp Phe Thr Leu Lys Ile Ser Arg
   65                  70                  75                  80
   Val Glu Ala Glu Asp Val Gly Val Tyr Tyr Cys Trp Gln Ser Thr His
                   85                  90                  95
   Phe Pro Trp Thr Phe Gly Gln Gly Thr Lys Val Glu Ile Lys Arg
               100                 105                 110
<210> 8
   <211> 111
   <212> PRT
   <213> Artificial Sequence
<220>
   <223> Synthetic
<400> 8
   Asp Ile Gln Met Thr Gln Ser Pro Leu Ser Leu Ser Val Thr Pro Gly
   1               5                   10                  15
   Gln Pro Ser Ile Ser Cys Lys Ser Ser Gln Leu Leu Asp Tyr Asp Gly
               20                  25                  30
   Lys Thr Tyr Leu Asn Trp Tyr Gln Gln Lys Pro Gly Gln Ser Pro Gln
           35                  40                  45
   Leu Leu Ile Tyr Gln Val Ser Lys Leu Asn Ser Gly Val Pro Asp Arg
       50                  55                  60
   Phe Ser Gly Ser Gly Ser Gly Thr Asp Phe Thr Leu Lys Ile Ser Arg
   65                  70                  75                  80
   Val Glu Ala Glu Asp Val Gly Val Tyr Tyr Cys Trp Gln Ser Thr His
   85 90 95
   Phe Pro Trp Thr Phe Gly Gln Gly Thr Lys Val Glu Ile Lys Arg
   100 105 110
<210> 9
   <211> 452
   <212> PRT
   <213> Artificial Sequence
<220>
   <223> Synthetic
<400> 9
   Glu Val Gln Leu Gln Glu Ser Gly Gly Gly Leu Val Gln Pro Gly Gly
   1 5 10 15
   Ser Leu Arg Leu Ser Cys Ala Ala Ser Gly Phe Thr Phe Ser Asp Ala
   20 25 30
   Trp Met Asp Trp Val Arg Gln Ala Pro Gly Lys Gly Leu Glu Trp Val
   35 40 45
   Gly Glu Ile Arg Ser Lys Ala Asn Asn Tyr Ala Thr Tyr Phe Ala Glu
   50 55 60
   Ser Val Lys Gly Arg Phe Thr Ile Ser Arg Asp Asp Ser Lys Asn Ser
   65 70 75 80
   Leu Tyr Leu Gln Met Asn Ser Leu Lys Thr Glu Asp Thr Gly Val Tyr
   85 90 95
   Tyr Cys Thr Lys Leu Ser Leu Arg Tyr Trp Phe Phe Asp Val Trp Gly
   100 105 110
   Gln Gly Thr Leu Val Thr Val Ser Ser Ala Ser Thr Lys Gly Pro Ser
   115 120 125
   Val Phe Pro Leu Ala Pro Ser Ser Lys Ser Thr Ser Gly Gly Thr Ala
   130 135 140
   Ala Leu Gly Cys Leu Val Lys Asp Tyr Phe Pro Glu Pro Val Thr Val
   145 150 155 160
   Ser Trp Asn Ser Gly Ala Leu Thr Ser Gly Val His Thr Phe Pro Ala
   165 170 175
   Val Leu Gln Ser Ser Gly Leu Thr Ser Leu Ser Ser Val Val Thr Val
   180 185 190
   Pro Ser Ser Ser Leu Gly Thr Gln Thr Tyr Ile Cys Asn Val Asn His
   195 200 205
   Lys Pro Ser Asn Thr Lys Val Asp Lys Lys Val Glu Pro Lys Ser Cys
   210 215 220
   Asp Lys Thr His Thr Cys Pro Pro Cys Pro Ala Pro Glu Leu Leu Gly
   225 230 235 240
   Gly Pro Ser Val Phe Leu Phe Pro Pro Lys Pro Lys Asp Thr Leu Met
   245 250 255
   Ile Ser Arg Thr Pro Glu Val Thr Cys Val Val Val Asp Val Ser His
   260 265 270
   Glu Asp Pro Glu Val Lys Phe Asn Trp Tyr Val Asp Gly Val Glu Val
   275 280 285
   His Asn Ala Lys Thr Lys Pro Arg Glu Glu Gln Tyr Asn Ser Thr Tyr
   290 295 300
   Arg Val Val Val Ser Val Leu Thr Val Leu His Gln Asp Trp Leu Asn
   305 310 315 320
   Gly Lys Glu Tyr Lys Cys Lys Val Ser Asn Lys Ala Leu Pro Ala Pro
   325 330 335
   Ile Glu Lys Thr Ile Ser Lys Ala Lys Gly Gln Pro Arg Glu Pro Gln
   340 345 350
   Val Tyr Thr Leu Pro Pro Ser Arg Asp Glu Leu Thr Lys Asn Gln Val
   355 360 365
   Ser Leu Thr Cys Leu Val Lys Gly Phe Tyr Pro Ser Asp Ile Ala Val
   370 375 380
   Glu Trp Glu Ser Asn Gly Gln Pro Glu Asn Asn Tyr Lys Thr Thr Pro
   385 390 395 400
   Pro Val Leu Asp Ser Asp Gly Ser Phe Phe Leu Tyr Ser Lys Leu Thr
   405 410 415
   Val Asp Lys Ser Arg Trp Gln Gln Gly Asn Val Phe Ser Cys Ser Val
   420 425 430
   Met His Glu Ala Leu His Asn His Tyr Thr Gln Lys Ser Leu Ser Leu
           435                 440                 445
   Ser Pro Gly Lys
       450
<210> 10
   <211> 217
   <212> PRT
   <213> Artificial Sequence
<220>
   <223> Synthetic
<400> 10
   Asp Ile Gln Met Thr Gln Ser Pro Leu Ser Leu Ser Val Thr Pro Gly
   1               5                   10                  15
   Gln Pro Ser Ile Ser Cys Lys Ser Ser Gln Leu Leu Asp Tyr Asp Gly
               20                  25                  30
   Lys Thr Tyr Leu Asn Trp Tyr Gln Gln Lys Pro Gly Gln Ser Pro Gln
           35                  40                  45
   Leu Leu Ile Tyr Gln Val Ser Lys Leu Asn Ser Gly Val Pro Asp Arg
       50                  55                  60
   Phe Ser Gly Ser Gly Ser Gly Thr Asp Phe Thr Leu Lys Ile Ser Arg
   65                  70                  75                  80
   Val Glu Ala Glu Asp Val Gly Val Tyr Tyr Cys Trp Gln Ser Thr His
                   85                  90                  95
   Phe Pro Trp Thr Phe Gly Gln Gly Thr Lys Val Glu Ile Lys Arg Thr
               100                 105                 110
   Val Ala Ala Pro Ser Val Phe Ile Phe Pro Pro Ser Asp Glu Gln Leu
           115                 120                 125
   Lys Ser Gly Thr Ala Ser Val Val Cys Leu Leu Asn Asn Phe Tyr Pro
       130                 135                 140
   Arg Glu Ala Lys Val Gln Trp Lys Val Asp Asn Ala Leu Gln Ser Gly
   145                 150                 155                 160
   Asn Ser Gln Glu Ser Val Thr Glu Gln Asp Ser Lys Asp Ser Thr Tyr
                   165                 170                 175
   Ser Leu Ser Ser Thr Leu Thr Leu Ser Lys Ala Asp Tyr Glu Lys His
               180                 185                 190
   Lys Val Tyr Ala Cys Glu Val Thr His Gln Gly Leu Ser Ser Pro Val
           195                 200                 205
   Thr Lys Ser Phe Asn Arg Gly Glu Cys
       210                 215
<210> 11
   <211> 1353
   <212> DNA
   <213> Artificial Sequence
<220>
   <223> Synthetic polynucleotide
<400> 11
   gaagtgcagc ttcaggagtc tggaggaggc ttggtgcagc ctggaggatc cctgcgcctc 60
   tcttgtgctg cctctggatt cacttttagt gacgcctgga tggactgggt ccgccaggct 120
   ccaggcaagg ggcttgagtg ggttggtgaa attagaagca aagctaataa ttatgcaaca 180
   tactttgctg agtctgtgaa agggaggttc accatctcaa gagatgattc caaaaatagt 240
   ctgtacctgc aaatgaacag cttaaaaact gaggacactg gcgtgtatta ctgtaccaag 300
   ttatcactac gttactggtt cttcgatgtc tggggccaag ggaccctggt caccgtctcc 360
   tcagctagca ccaagggccc atcggtcttc cccctggcac cctcctccaa gagcacctct 420
   gggggcacag cggccctggg ctgcctggtc aaggactact tccccgaacc ggtgacggtg 480
   tcgtggaact caggcgccct gaccagcggc gtgcacacct tcccggctgt cctacagtcc 540
   tcaggactct actccctcag cagcgtggtg accgtgccct ccagcagctt gggcacccag 600
   acctacatct gcaacgtgaa tcacaagccc agcaacacca aggtggacaa gaaagtggag 660
   cccaaatctt gtgacaaaac tcacacatgc ccaccgtgcc cagcacctga actcctgggg 720
   ggaccgtcag tcttcctctt ccccccaaaa cccaaggaca ccctcatgat ctcccggacc 780
   cctgaggtca catgcgtggt ggtggacgtg agccacgaag accctgaggt caagttcaac 840
   tggtacgtgg acggcgtgga ggtgcataat gccaagacaa agccgcggga ggagcagtac 900
   aacagcacgt accgtgtggt cagcgtcctc accgtcctgc accaggactg gctgaatggc 960
   aaggagtaca agtgcaaggt ctccaacaaa gccctcccag cccccatcga gaaaaccatc 1020
   tccaaagcca aagggcagcc ccgagaacca caggtgtaca ccctgccccc atcccgggat 1080
   gagctgacca agaaccaggt cagcctgacc tgcctggtca aaggcttcta tcccagcgac 1140
   atcgccgtgg agtgggagag caatgggcag ccggagaaca actacaagac cacgcctccc 1200
   gtgctggact ccgacggctc cttcttcctc tacagcaagc tcaccgtgga caagagcagg 1260
   tggcagcagg ggaacgtctt ctcatgctcc gtgatgcatg aggctctgca caaccactac 1320
   acgcagaaga gcctctccct gtctccgggt aaa 1353
<210> 12
   <211> 657
   <212> DNA
   <213> Artificial Sequence
<220>
   <223> Synthetic polynucleotide
<400> 12
   gatttcgtca tgacccagag cccactctct ctgtccgtca cccctggaca gccagcctcc      60
   atctcttgca agtcaagtca gagcctcttg gattacgatg gaaagacata tttgaattgg     120
   ttgcagcaga agccaggcca gtctccaaaa catctcatct atcaggtgtc taaactgaac     180
   tctggagtcc ctgacaggtt cagtggcagt ggatcaggga cagatttcac actgaaaatc     240
   agccgggtgg aggctgagga tgtgggggtt tattactgct ggcaaagtac acattttccg     300
   tggacgttcg gccaggggac caaggtggaa atcaaacgga cggtggctgc accatctgtc     360
   ttcatcttcc cgccatctga tgagcagttg aaatctggaa ctgcctctgt tgtgtgcctg     420
   ctgaataact tctatcccag agaggccaaa gtacagtgga aggtggataa cgccctccaa     480
   tcgggtaact cccaggagag tgtcacagag caggacagca aggacagcac ctacagcctc     540
   agcagcaccc tgacgctgag caaagcagac tacgagaaac acaaagtcta cgcctgcgaa     600
   gtcacccatc agggcctgag ctcgcccgtc acaaagagct tcaacagggg agagtgt        657
<210> 13
   <211> 121
   <212> PRT
   <213> Artificial Sequence
<220>
   <223> Synthetic
<400> 13
   Glu Val Gln Leu Gln Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ala
               20                  25                  30
   Trp Met Asp Trp Val Arg Gln Ala Pro Gly Lys Gly Leu Glu Trp Val
           35                  40                  45
   Gly Glu Ile Arg Ser Lys Ala Asn Asn Tyr Ala Thr Tyr Phe Ala Glu
       50                  55                  60
   Ser Val Lys Gly Arg Phe Thr Ile Ser Arg Asp Asp Ser Lys Asn Ser
   65 70 75 80
   Leu Tyr Leu Gln Met Asn Ser Leu Lys Thr Glu Asp Thr Gly Val Tyr
   85 90 95
   Tyr Cys Thr Lys Leu Ser Leu Arg Tyr Trp Phe Phe Asp Val Trp Gly
   100 105 110
   Gln Gly Thr Leu Val Thr Val Ser Ser
   115 120
<210> 14
   <211> 113
   <212> PRT
   <213> Artificial Sequence
<220>
   <223> Synthetic
<400> 14
   Asp Ile Gln Met Thr Gln Ser Pro Leu Ser Leu Ser Val Thr Pro Gly
   1 5 10 15
   Gln Pro Ala Ser Ile Ser Cys Lys Ser Ser Gln Ser Leu Leu Asp Tyr
   20 25 30
   Asp Gly Lys Thr Tyr Leu Asn Trp Tyr Gln Gln Lys Pro Gly Gln Ser
   35 40 45
   Pro Gln Leu Leu Ile Tyr Gln Val Ser Lys Leu Asn Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Trp Gln Ser
   85 90 95
   Thr His Phe Pro Trp Thr Phe Gly Gln Gly Thr Lys Val Glu Ile Lys
   100 105 110
   Arg
<210> 15
   <211> 120
   <212> PRT
   <213> Homo sapiens
<400> 15
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Asn Ile Lys Asp Thr
               20                  25                  30
   Tyr Ile His Trp Val Arg Gln Ala Pro Gly Lys Gly Leu Glu Trp Val
           35                  40                  45
   Ala Arg Ile Tyr Pro Thr Asn Gly Tyr Thr Arg Tyr Ala Asp Ser Val
       50                  55                  60
   Lys Gly Arg Phe Thr Ile Ser Arg Asp Asp Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ser Arg Trp Gly Gly Asp Gly Phe Tyr Ala Met Asp Val Trp Gly Gln
               100                 105                 110
   Gly Thr Leu Val Thr Val Ser Ser
           115                 120
<210> 16
   <211> 121
   <212> PRT
   <213> Artificial Sequence
<220>
   <223> Synthetic
<400> 16
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asp Ala
               20                  25                  30
   Trp Met Asp Trp Val Arg Gln Ala Pro Gly Lys Gly Leu Glu Trp Val
           35                  40                  45
   Ala Glu Ile Arg Ser Lys Ala Asn Asn Tyr Ala Thr Tyr Phe Ala Glu
       50                  55                  60
   Ser Val Lys Gly Arg Phe Thr Ile Ser Arg Asp Asp Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Thr Lys Leu Ser Leu Arg Tyr Trp Phe Phe Asp Val Trp Gly
               100                 105                 110
   Gln Gly Thr Leu Val Thr Val Ser Ser
           115                 120
<210> 17
   <211> 108
   <212> PRT
   <213> Homo sapiens
<400> 17
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Asn Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Glu Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln His Tyr Thr Thr Pro Pro
                   85                  90                  95
   Thr Phe Gly Gln Gly Thr Lys Val Glu Ile Lys Arg
               100                 105
<210> 18
   <211> 113
   <212> PRT
   <213> Artificial Sequence
<220>
   <223> Synthetic
<400> 18
   Asp Phe Val Met Thr Gln Ser Pro Ser Ser Leu Ser Ala Ser Val Gly
   1               5                   10                  15
   Asp Arg Val Thr Ile Thr Cys Lys Ser Ser Gln Ser Leu Leu Asp Tyr
               20                  25                  30
   Asp Gly Lys Thr Tyr Leu Asn Trp Leu Gln Gln Lys Pro Gly Lys Ala
           35                  40                  45
   Pro Lys His Leu Ile Tyr Gln Val Ser Lys Leu Asn Ser Gly Val Pro
       50                  55                  60
   Ser Arg Phe Ser Gly Ser Gly Ser Gly Thr Asp Phe Thr Leu Thr Ile
   65                  70                  75                  80
   Ser Ser Leu Gln Pro Glu Asp Phe Ala Thr Tyr Tyr Cys Trp Gln Ser
                   85                  90                  95
   Thr His Phe Pro Trp Thr Phe Gly Gln Gly Thr Lys Val Glu Ile Lys
               100                 105                 110
   Arg
<210> 19
   <211> 5
   <212> PRT
   <213> Mus musculus
<400> 19
   Asp Ala Trp Met Asp
   1               5
<210> 20
   <211> 19
   <212> PRT
   <213> Mus musculus
<400> 20
   Glu Ile Arg Ser Lys Ala Asn Asn Tyr Ala Thr Tyr Phe Ala Glu Ser
   1               5                   10                  15
   Val Lys Gly
<210> 21
   <211> 10
   <212> PRT
   <213> Mus musculus
<400> 21
   Leu Ser Leu Arg Tyr Trp Phe Phe Asp Val
   1               5                   10
<210> 22
   <211> 16
   <212> PRT
   <213> Mus musculus
<400> 22
   Lys Ser Ser Gln Ser Leu Leu Asp Ser Asp Gly Lys Thr Tyr Leu Asn
   1               5                   10                  15
<210> 23
   <211> 7
   <212> PRT
   <213> Mus musculus
<400> 23
   Leu Val Ser Lys Leu Asp Ser
   1               5
<210> 24
   <211> 9
   <212> PRT
   <213> Mus musculus
<400> 24
   Trp Gln Ser Thr His Phe Pro Trp Thr
   1               5

## Description

### BACKGROUND OF THE INVENTION

### Field of the Invention

The field of the present invention is humanized antibodies against interleukin-20.

### Background

Interleukin-20 (hereinafter "IL-20") belongs to the IL-10 family. IL-20 is a pleiotropic cytokine with chemoattractive, angiogenic, and osteoclastogenesis characteristics and plays an important role in various inflammation diseases and other disorders. Evidence suggesting IL-20 involvement in various pathological conditions include: up-regulation of both IL-20 and its receptors in atherosclerosis lesions, in synovial fluid of rheumatoid arthritis (RA) patients, and in human atherosclerotic artery. Furthermore, IL-20 is shown to promote atherosclerosis in apolipoprotein E-deficient mice, to induce neutrophil chemotaxis, to induce rheumatoid arthritis (RA) synovial fibroblasts (RASF) migration, and to promote angiogenesis. See e.g., U.S. Patent No. 7,786,274, issued to Chang.

Inhibition of IL-20 functions has been shown to prevent or reduce the developments of these diseases or disorders. For example, IL-20 receptor I (as a decoy receptor) has been shown to inhibit collagen-induced arthritis. Therefore, anti-IL-20 reagents (such as antibodies against IL-20) can be promising therapeutic options for the treatments and managements of IL-20-induced diseases.

U.S. Patent No. 7,786,274, issued to Chang, discloses a monoclonal antibody against IL-20 (mAb 7E). This monoclonal antibody (mAb 7E) is a mouse mAb, generated by immunizing BALB/cJ mice with recombinant hIL-20. This mAb 7E has been shown to be useful in treating various IL-20 associated inflammatory diseases, such as rheumatoid arthritis (RA), psoriasis, psoriatic arthritis, bacteria-induced gastric ulcer, and acute renal failure.

For Example, U.S. Patent No. 7,611,705 shows that mAb 7E can be used to treat IL-20 associated inflammatory diseases. U.S. Patent Nos. 7,837,994 and 8,454,956 show that mAb 7E can be used to treat osteoporosis. U.S. Patent No. 8,012,478 shows that mAb 7E can be used to treat ischemic stroke.

These prior art results show that anti-IL-20 antibodies (e.g., mAb 7E) can be useful therapeutic agents for the treatments and control of various IL-20 associated diseases. However, these antibodies are mouse antibodies, which may elicit immune responses when used as therapeutic agents. Such immune responses pose major limitations for the use of such antibodies in clinics. Recently, U.S. Patent No. 8,597,647, issued to Chang et aL, disclosed a humanized analog of mAb 7E, which was produced by grafting all six CDR sequences from mAb 7E into a human heavy chain and a light chain variable sequences. The resultant humanized antibodies have lower affinities for IL-20, as compared with the original mAb 7E.

All these prior art results indicate that anti-IL-20 antibodies can be useful. However, there is still a need for better anti-IL-20 antibodies.

### SUMMARY OF THE INVENTION

Embodiments of the present invention relate to anti-IL-20 antibodies or fragments thereof. The antibodies may be monoclonal antibodies, humanized antibodies, human antibodies, chimeric antibodies, or fragments thereof. Fragments of such antibodies may include Fab, scFv, F(ab')₂, etc. Antibodies of the invention are generated based on human immunoglobulin heavy chain and light chain sequences, the CDR sequences of which are mutated to those of a known anti-IL-20 antibody from other animals (e.g., mouse) that bind tightly to IL-20 (e.g., mAb 7E disclosed in U.S. Patent No. 7,786,274). Such initial humanized antibodies may be further optimized to afford antibodies with substantially higher affinities for IL-20 and/or lower immunogenicities when used as therapeutics, as compared with the original mouse anti-IL-20 antibody.

One aspect of the invention relates to humanized antibodies against IL-20, or a scFv, Fab, or F(ab')₂ fragments thereof. In accordance with one embodiment of the invention, a humanized antibody against IL-20, or a scFv, Fab, or F(ab')₂ fragment thereof, includes a heavy chain variable region comprising SEQ ID NO:4; and a light chain variable region comprising SEQ ID NO:8.

In accordance with one embodiment of the invention, an antibody is FLB5M5 that comprises a heavy chain variable region having the sequence of SEQ ID NO: 4 and a light chain variable region having the sequence of SEQ ID NO: 8. The FLB5M5 antibody may contain the constant regions from human immunoglobulin. For example, FLB5M5 may have a full heavy chain having the sequence shown in SEQ ID NO:9, and a full light chain having the sequence shown in SEQ ID NO: 10, and the corresponding coding sequences are shown in SEQ ID NO: 11 and SEQ ID NO: 12, respectively.

In any of the above embodiments of the present invention, the anti-IL-20 antibodies may include humanized monoclonal antibodies, such as FLB5M5 (also referred to as hFLB5M5). FLB5 5 is a humanized monoclonal antibody with three mutated amino acids m the complementarity determining regions (CDRs), relative to its parental mouse anti-IL-20 monoclonal antibody 7E (mAb 7E), and five mutated amino acids of the light-chain framework region, relative to the amino acids of the light-chain framework region of human V 2. FLB5M5 not only retains binding specificity toward IL-20 but also has an unexpected better binding affinity than mAb 7E for IL-20. FLB5M5 is also less immunogenic than mAb 7E to the human host in clinical application.

A further embodiment is defined in the dependent claim.

A further aspect of the invention relates to a composition comprising the humanized antibody against IL-20, or a scFv, Fab, or F(ab')₂ fragment thereof, as defined in any of the above-mentioned embodiments for use in therapy.

The IL-20 associated disease is one or more selected from the group consisting of rheumatoid arthritis, osteoporosis, cancer, stroke, and renal failure.

### BRIEF DESCRIPTION OF TFTE DRAWINGS

FIG. 1 shows sequence comparison of the amino acids of mAb 7E (SEQ ID NO:1), human homolog (IGHV3-72*01) (SEQ ID NO:2), HH12 (SEQ ID NO:3), and FLB5M5 (SEQ ID NO:4), in the heavy chain variable regions (VH regions).
FIG. 2 shows sequence comparison of the amino acids of mAb 7E (SEQ ID NO:5), human homolog (IGKV2D-29*02) (SEQ ID NO:6), HH12 (SEQ ID NO:7), and FLB5M5 (SEQ ID NO:8), in the light chain variable regions (VL regions).
FIG. 3 shows sequence comparison of the amino acids of mAb 7E (SEQ ID NO:1), human homolog (IGHV3-66*04) (SEQ ID NO:15), and DD10B5 (SEQ ID NO: 16), in the heavy chain variable regions (VH regions). DD10B5 is a humanized antibody having a heavy chain variable region based on IGHV3-66*04.
FIG. 4 shows sequence comparison of the amino acids of mAb 7E (SEQ ID NO:5), human homolog (IGKV1-39*01) (SEQ ID NO: 17), and DD10B5 (SEQ ID NO:18), in the light chain variable regions (V_{L} regions). DD10B5 is a humanized antibody having a heavy chain variable region based on IGKV1-39*01.
FIG. 5 shows summary of sequence comparisons between the amino acids of mAb 7E and the various human homologs shown in FIGs. 1-4.
FIG. 6 shows results of inhibition of IL-20-induced proliferation by various antibodies: mAb E7, FLB5, and FLB5M5.
FIG. 7A shows results from prevention of arthritis (measured with arthritis score (AS)) by treatments with different doses (1 mg/kg, 3 mg/kg, or 9 mg/kg) of hFLB5M5 and compared with treatment using Enbrel (6 mg/kg) as a positive control, in a rat rheumatoid arthritis model.
FIG. 7B shows results from prevention of arthritis (measured with hind-paw thickness) by treatments with different doses (1 mg/kg, 3 mg/kg, or 9 mg/kg) of hFLB5M5 and compared with treatment using Enbrel (6 mg/kg) as a positive control, in a rat rheumatoid arthritis model.
FIG. 7C shows results from prevention of arthritis (measured with body weight) by treatments with different doses (1 mg/kg, 3 mg/kg, or 9 mg/kg) of hFLB5M5 and compared with treatment using Enbrel (6 mg/kg) as a positive control, in a rat rheumatoid arthritis model.
FIG. 8 shows results of alanine substitutions for three important residues in CDRH3 and CDRL3 of HH12 and FLB5M5. The results show that W100 in CDRH3 is important because alanine substitution at this position substantially reduce the binding affinities of both antibodies (HH12 and FLB5M5). In CDRL3, F94 seems to be more important than Q90.

### Definition

Unless otherwise defined, scientific and technical terms used herein shall have the meanings that are commonly understood by those of ordinary skill in the art. Furthermore, unless otherwise required by context, singular terms shall include pluralities and plural terms shall include the singular. A list of abbreviations used herein is as follows: Ab: antibody; BaF3: murine precursor B cells; CDRs: complementarity determining regions; C_{H}: heavy-chain constant domain; C_{L}: light-chain constant domain; HEPES: 4-(2-hydroxyethyl)-1-piperazineethanesulfonic acid; IL-20: interleukin-20; LTM: look-through mutagenesis; PBS: phosphate buffered saline; PCR: polymerase chain reaction; PTH: hind paw thickness; AS: arthritis score; RPMI medium: Roswell Park Memorial Institute medium; scFv: single-chain variable fragment; SD rats: Sprague-Dawley rats; V_{L}: light-chain variable domain; V_{H}: heavy-chain variable domain.

Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. These techniques and procedures are generally performed according to conventional methods well known in the art and as described in various general and more specific references that are cited and discussed throughout the present specification. See e.g., Sambrook et al., Molecular Cloning: A Laboratory Manual (3rd ed., Cold Spring Harbor Laboratory Press, Cold Spring Harbor, N.Y. (2001).

As used herein, the term "antibody" refers generally and broadly to immunoglobulins, monoclonal antibodies, and polyclonal antibodies, as well as active fragments thereof. The fragment may be active in that it binds to the cognate antigen (i.e., IL-20 or fragment of IL-20), or it may be active in that it is biologically functional. The antibodies of the invention may be chimeric, humanized, or human, using techniques known in the art.

As used herein, the term "humanized antibody" refers to an antibody in which minimal portions of a non-human antibody are introduced into an otherwise human antibody.

As used herein, the term "human antibody" refers to an antibody in which substantially every part of the protein is substantially non-immunogenic in humans, with only minor sequence changes or variations.

An antigen binding site is generally formed by the heavy chain variable region (VH) and the light chain variable region (VL) immunoglobulin domains, with the antigen-binding interface formed by six surface polypeptide loops, termed complimentarity determining regions (CDRs). There are three CDRs each in VH (HCDR1, HCDR2, HCDR3) and VL (LCDR1, LCDR2, LCDR3), together with framework regions (FRs).

The term "CDR region" or "CDR" means the hypervariable regions of the heavy or light chains of the immunoglobulin as defined by Kabat et al., 1991 (Kabat, E. A. et al., (1991) Sequences of Proteins of Immunological Interest, 5th Edition. US Department of Health and Human Services, Public Service, NIH, Washington), and later editions. An antibody typically contains 3 heavy chain CDRs and 3 light chain CDRs.

The term a "set of CDRs" referred to herein comprises CDR1, CDR2 and CDR3. Thus, a set of HCDRs refers to HCDR1, HCDR2 and HCDR3 (HCDR refers to a variable heavy chain CDR), and a set of LCDRs refers to LCDR1, LCDR2 and LCDR3 (LCDR refers to a variable light chain CDR). Unless otherwise stated, a "set of CDRs" includes HCDRs and LCDRs.

It has been shown that fragments of a whole antibody can also bind antigens. Examples of binding fragments include: (i) an Fab fragment consisting of VL, VH, CL and CHl domains (Ward, E. S. et al., (1989) Nature 341, 544-546); (ii) an Fd fragment consisting of the VH and CH1 domains (McCafferty et al., (1990) Nature, 348, 552-554); (iii) an Fv fragment consisting of the VL and VH domains of a single antibody (Holt et al., (2003) Trends in Biotechnology 21, 484-490); (iv) a dAb fragment (Ward, E. S. et al., Nature 341, 544-546 (1989), McCafferty et al., (1990) Nature, 348, 552-554, Holt et al., (2003) Trends in Biotechnology 21, 484-490], which consists of a VH or a VL domain; (v) isolated CDR regions; (vi) F(ab')₂ fragments, a bivalent fragment comprising two linked Fab fragments (vii) single chain Fv molecules (scFv), wherein a VH domain and a VL domain are linked by a peptide linker which allows the two domains to associate to form an antigen binding site (Bird et al., (1988) Science, 242, 423-426, Huston et al., (1988) PNAS USA, 85, 5879-5883); (viii) bispecific single chain Fv dimers (PCT/US92/09965) and (ix) "diabodies", multivalent or multispecific fragments constructed by gene fusion (WO94/13804; Holliger, P. (1993) et al., Proc. Natl. Acad. Sci. USA 90 6444-6448).

Fv, scFv or diabody molecules may be stabilized by incorporation of disulfide bridges linking the VH and VL domains (Reiter, Y. et al., Nature Biotech, 14, 1239-1245, 1996). Minibodies comprising a scFv joined to a CH3 domain may also be made (Hu, S. et al., (1996) Cancer Res., 56, 3055-3061). Other examples of binding fragments are Fab', which differs from Fab fragments by the addition of a few residues at the carboxyl terminus of the heavy chain CH1 domain, including one or more cysteines from the antibody hinge region, and Fab'-SH, which is a Fab' fragment in which the cysteine residue(s) of the constant domains bear a free thiol group.

Digestion of antibodies with the enzyme, papain, results in two identical antigen-binding fragments, known also as "Fab" fragments, and a "Fc" fragment, having no antigen-binding activity but having the ability to crystallize. Digestion of antibodies with the enzyme, pepsin, results in the a F(ab')₂ fragment in which the two arms of the antibody molecule remain linked and comprise two-antigen binding sites. The F(ab')₂ fragment has the ability to crosslink antigen.

"Fv" when used herein refers to the minimum fragment of an antibody that retains both antigen-recognition and antigen-binding sites. "Fab" when used herein refers to a fragment of an antibody that comprises the constant domain of the light chain and the CH1 domain of the heavy chain. The term "mAb" refers to monoclonal antibody.

### DETAILED DESCRIPTION OF THE INVENTION

Embodiments of the invention relate to humanized anti-IL-20 antibodies or fragments thereof. The antibodies of the invention are monoclonal antibodies generated using molecular biology techniques. Fragments of these antibodies may include Fab, scFv, F(ab')₂, chimeric antibodies, etc. As compared with mAb 7E disclosed in U.S. Patent No. 7,786,274, antibodies of the invention are exhibit higher affinities for IL-20 and/or lower immunogenicities when used as therapeutics.

Because a major limitation in the use of non-human antibodies in therapy is that these antibodies may elicit undesired immune responses, inventors of the present invention set out to design better anti-IL-20 antibodies by starting from human immunoglobulin sequences. Specifically, antibodies of the invention are produced starting with human framework sequences. These human antibody framework sequences are then modified to have IL-20 binding affinities. The modifications involve changing the sequences in the complementarity-determining regions (CDRs). Any methods known in the art may be used to produce the IL-20 binding sites, including random mutagenesis and screening, phage display and panning, or grafting of CDR sequences from known anti-IL-20 antibodies.

In accordance with embodiments of the invention, for example, selected immunoglobulin sequences may be endowed with IL-20 binding affinities by incorporating CDR sequences from known anti-IL-20 antibodies, such as mAb 7E. Using mAb 7E as an example, the following describes some specific examples for the production of antibodies having human framework sequences but containing mAb 7E CDR sequences. However, one skilled in the art would appreciated that similar antibodies may be produced using CDR sequences from other anti-IL-20 antibodies. The immediate products from the CDR grafting are chimeric antibodies, which contain human framework sequences and CDR sequences from another sources (e.g., mouse sequences).

Using the CDR grafting approach, it would be better to start with human antibody framework sequences that are homologous to the framework sequences of the antibody that will provide the CDR sequences. Such homologous sequences may be identified using consensus or homology searches with a known anti-IL-20 antibody framework sequence as a query sequence. A sequence with high homology is likely to provide the same framework structures to preserve the binding affinities of the target CDR sequences. Briefly, variable light (VL) and variable heavy (VH) domain framework sequences from human immunoglobulin subgroups having high homology with a target antibody (e.g., mAb 7E) may be identified as starting points.

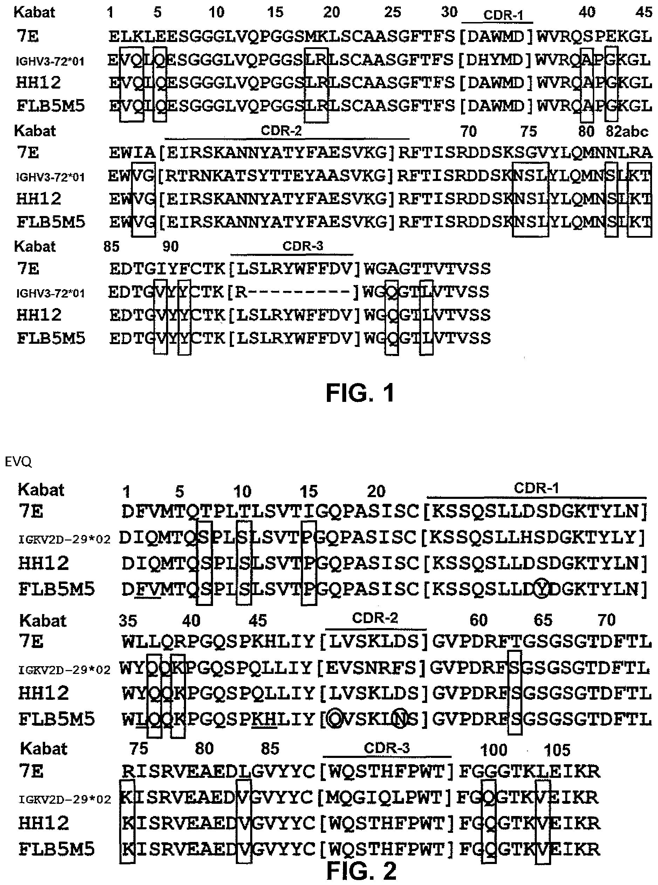

In accordance with embodiments of the invention, the heavy chain framework sequences may be from VH subgroup III, and the light chain framework sequences may be from VL kappa subgroup II (V_{K}2). For example, in accordance with one embodiment of the invention, the VH subgroup II sequence that is highly homologous to the framework regions of mAb 7E is found to be IGHV3-72*01, and the Vκ2 sequence that is highly homologous to the corresponding framework regions of mAb 7E is found to be IGKV2D-29*02. The sequence comparison between IGHV3-72*01 and the heavy chain variable region of mAb 7E is shown in FIG. 1, while the sequence comparison between IGKV2D-29*02 and the light chain variable region of mAb 7E is shown in FIG. 2. In FIG. 1 and FIG. 2, the sequence differences in the framework regions (i.e., non-CDR regions) are shown as boxed residues.

Based on the IGHV3-72*01 and the IGKV2D-29*02 sequences, the CDR sequences (i.e., CDR-1, CDR-2, and CDR-3) in each of which may be converted to the corresponding CDR sequences of the target anti-IL-20 antibody (e.g., mAb 7E). These conversions would generate a chimeric heavy chain and a chimeric light chain shown in FIG. 1 and FIG. 2, respectively. These chimeric heavy chain and light chain may be used to construct an scFv or Fab fragment, using techniques known in the art. Alternatively, the human framework sequences and the mouse CDR sequences may be assembled into an scFv or Fv fragment in a single step using overlap PCR, as described in the following example.

In yet another alternative approach, an scFv or Fab fragment may be constructed first, based on the identified human framework sequences for the VH and VL regions (e.g., IGHV3-72*01 and the IGKV2D-29*02 sequences). Then, such an scFv or Fab fragment may then be modified into a humanized anti-IL-20 antibody, by mutating the CDR sequences in the Fab fragment into known CDR sequences of a target anti-IL-20 antibody (e.g., mAb 7E) or to sequences substantially the same as the known CDR sequences of the target anti-IL-20 antibody.

As noted above, the mutations or CDR grafting may be accomplished with any methods known in the art, for example by site directed mutagenesis or by PCR incorporation of mutated residues/fragments (e.g., overlap PCR).

As an example, an scFv fragment based on IGHV3-72*01 as a heavy chain variable sequence and IGKV2D-29*02 as a light chain variable sequence and containing all six CDR sequences from mAb 7E is a humanized anti-IL-20 antibody, HH12, the heavy chain and light chain variable regions of which are shown in FIG. 1 and FIG. 2, respectively.

In accordance with embodiments of the invention, the initial chimeric antibodies (such as HH12) may be further modified to improve their affinities for IL-20. These modifications may be referred to as affinity maturation, which may be accomplished with techniques known in the art, such as random mutagenesis and screening, phage display and panning, etc. These affinity maturations may involve residues in the CDR regions and/or non-CDR regions.

As an example, an scFv fragment, FLB5M5 (also referred to as hFLB5M5), was obtained by affinity maturation of HH12. FLB5M5 contains three amino acid mutations in the complementarity determining regions (CDRs), as compared to the starting HH12 antibody or the parental mouse anti-IL-20 monoclonal antibody 7E (mAb 7E). These three amino acid mutations are indicated with circles in FIG. 2.

In addition, FLB5M5 contains five amino acid mutations in the light-chain framework region, as compared to the amino acids of the light-chain framework region of human Vκ2. The five amino acid mutations in the framework regions are illustrated as underlined residues in FIG. 2. The mutations in the non-CDR regions (i.e., the framework regions) may be by random mutagenesis. Alternatively, one may selectively replace the amino acid residues in the framework regions that differ from the corresponding amino acids in the "model" mouse antibody (e.g., mAb 7E) with those amino acids in the original "model" antibody. The rationale for putting the different amino acids back to what were in the original antibody (referred to as "back mutation") is that such amino acids in the framework regions may indirectly impact the binding of the antigen due to minor conformational changes in the paratope regions. The five amino acid mutations in the framework regions in FLB5M5 result from such "back mutations."

FLB5M5 represents an example of a product of affinity maturation by mutating amino acids in the light chain variable regions. One skilled in the art would appreciate that other affinity matured antibodies may be similarly obtained.

Even though FLB5M5 is an artificially generated antibody, it was surprisingly found that it not only exhibits the binding specificity for IL-20, but also has a better binding affinity than that of natural mAb 7E. In addition, because FLB5M5 contains mostly human immunoglobulin sequences, it is expected that this humanized monoclonal antibody would elicit less immune response in a human host. Thus, FLB5M5 and similar humanized antibodies are promising therapeutics for clinical applications.

In an attempt to understand the underlying mechanism for the enhanced affinity of FLB5M5, a structure-activity relationship (SAR) study was performed. Such SAR studies may be conducted using any techniques known in the art, such as alanine scanning.

From such an SAR study, it was found that a tyrosine residue (Y32) located in the light-chain variable domain CDR1 plays an important role for the increased IL-20 affinity in FLB5M5. In addition, it was found that the combination of this tyrosine with other amino acid residues mutation enhance the binding affinity.

Some embodiments of the present invention relate to methods for the production of humanized antibodies of the invention. A method for the production of a humanized antibody may include the steps of: (1) obtaining a sequence of a mouse monoclonal antibody (e.g., mAb 7E) as a model, based on which a humanized antibody is to be designed; (2) selecting human framework donor antibody sequences based on homology with the "model" mouse monoclonal antibody; and (3) replacing the sequences of the CDR regions in the framework donor antibody sequences with the corresponding CDR sequences from the mouse monoclonal antibody. In accordance with some embodiments, the method may further include: affinity maturation steps, which may include mutations of the amino acids in the CDR regions and/or replacing amino acids in the framework regions in order to enhance the antibody affinities. The mutations in the framework regions may be by replacing amino acids with those from the "model" mouse monoclonal antibody.

For example, in preparing a humanized antibody, the amino acid sequence of a model mouse monoclonal antibody (e.g., mAb 7E) may be obtained from the database or determined using methods known in the art. The amino acid sequence of mAb 7E may then be used to search for human germ-line V_{L} and V_{H} sequences with the highest degree of homology with the mAb 7E framework regions. The homology search can be performed using any methods known in the art, such as BLAST (basic local alignment search tool) available at the National Center for Biotechnology Information (NCBI).

The search for human homologs of mAb 7E found IGHV3-72*01 to be the homolog for the V_{H} of mAb 7E and IGKV2D-29*02 to be the homolog for V_{L} of mAb 7E. The sequence alignments between mAb 7E and these two sequences are shown in FIG. 1 (V_{H} regions) and FIG. 2 (V_{L} regions). In FIG. 1 and FIG. 2, the amino acids in the framework regions that are different between mAb 7E and the human homolog sequences (IGHV3-72*01 and IGKV2D-29*02) are shown in boxes. As can be seen from the sequence alignments, the differences in the framework regions are relatively minor, suggesting that these homolog sequences could be good candidates for antibody humanization.

Therefore, these two human homolog sequences are used as base sequences for the production of a humanized antibody. In one example, the CDR regions of the human homologs (IGHV3-72*01 for the V_{H} and IGKV2D-29*02 for V_{L}) were replaced with the corresponding CDR regions of mAb 7E by methods known in the art, such as by site-directed mutagenesis or by CDR grafting. CDR grafting, for example, may be accomplished by obtaining the oligonucleotide sequences of the cDR regions (e.g., by PCR or chemical synthesis) followed by ligation and/or cloning into suitable vectors to join with the remaining sequences of the framework regions. The CDR replacements produced a humanized anti-IL-20 antibody, HH12. As shown in FIG. 1 and FIG. 2, HH12 contains all six CDR sequences from the mouse mAB 7E, while retaining all human framework sequences.

Once HH12 was prepared, it was further modified to optimize the binding affinity (i.e., affinity maturation) for IL-20. In addition, further mutations may be performed to minimize its immunogenic effect in human hosts. First, the binding affinity of HH12 was optimized by mutations in the CDR regions. The mutations may be accomplished with random mutations at the selected sites (e.g., residues in the CDRL1, CDRL2, or CDRL3) followed by selection for the tight binders. Multiple methods of introducing mutations in the CDRs are known in the art, including radiation, chemical mutagens, and error-prone PCR. After such mutations, the mutants are screened for their binding affinities. Those with tighter bindings are selected and may be subjected to further runs of affinity maturations. This optimization process produced a new antibody that contains three mutations shown with circles in FIG. 2.

The antibody with optimized CDR sequences may be further enhanced by putting some of the framework amino acids in the human sequences back to the amino acids in the mouse sequences. Even though framework regions are not directly involved in antigen binding, they may indirectly impact the binding due to conformational effects. As shown in FIG. 2, five amino acids (underlined in FIG. 2) in the framework regions of FLB5M5 were mutated back to those found in the mouse mAB 7E sequences. These modifications can be performed in either order or simultaneously, i.e. modifying the framework first and then the CDRs or modifying the CDRs first and then the framework.

After these mutations/optimizations, a better antibody FLB5M5 was identified. In FLB5M5, five amino acids from the HH12 light-chain framework region were replaced with amino acids from the mouse monoclonal antibody 7E (i.e., I2F, Q3V, Y36L, Q45K, and L46H), and three amino acids in light-chain CDR1 and CDR2 were mutated (i.e., S32Y, L50Q, and D55N).

FIG. 1 and FIG. 2 summarize the alignments of the V_{H} and V_{L}, respectively, amino acid sequences among mAb 7E, the human homologs (IGHV3-72*01 for the V_{H} and IGKV2D-29*02 for V_{L}), HH12, and FLB5M5. In FIG. 2, amino acids that differ between FLB5M5 and HH12 in the CDR region are enclosed in rectangles and those in the framework regions are underlined.

Three mutations in the CDRs (circled residues in the CDR regions in FIG. 2) were found to be beneficial from the affinity maturation, with one mutation (from serine to tyrosine) in light chain CDR-1 found to be especially beneficial.

Table 1 shows IL-20 binding characteristics (*kₐ*: association rate; *k_{d}*: dissociation rate; and *K_{D}*: dissociation constant (i.e., inverse of affinity)) for several mutants in the CDR regions arising from affinity maturations. These binding parameters may be obtained using any methods known in the art, such as ELISA or BIACore binding assays described below.

**Table 1. Comparison of binding kinetics for various specimens with mutations in the CDRs**

| **Specimen** | **CDRH3** | | **CDRL1** | **CDRL2** | **CDRL3** | **kₐ** | **k_{d}** | **K_{D}** |
|---|---|---|---|---|---|---|---|---|
| | 96 | 98 | 27E | 50 | 93 | (M⁻¹ s⁻¹) | (s⁻¹) | (M) |
| HH12 | S | R | S | L | H | 1.34E+6 | 3.58E-3 | 2.68E-9 |
| G3L | S | R | Y | L | H | 8.12E+5 | 1.07E-3 | 1.31E-9 |
| FLB67 | S | Q | Y | Q | H | 8.88E+5 | 7.13E-4 | 8.03E-10 |
| FLB35 | D | R | Y | L | L | 9.88E+5 | 4.31E-4 | 4.37E-10 |
| FLB35(S) | D | R | S | L | L | 7.03E+5 | 2.83E-3 | 4.03E-9 |

From the data in Table 1, it can be concluded that mutations in CDRH3 did not produce much effects in the IL-20 binding affinity. On the other hand, mutation of serine-32 to tyrosine-32 in CDRL1 (i.e., 27E position in CDRL1), significantly improved the IL-20 binding affinities.

Back mutation identified five amino acids within the light-chain framework regions of HH12 that can increase the binding affinity to IL-20. In FIG. 2, these amino acids are underlined.

Finally, a combination of three mutations in the CDRs and five mutations within the light-chain framework region were all expressed as one humanized anti-IL-20 antibody, FLB5M5.

Anti-IL-20 antibodies (e.g., mAb 7E) have been shown to be effective in treating or preventing various IL-20 associated diseases, including osteoporosis (U.S. Patent Nos. 7,837,994 and 8,454,956), ischemic stroke (U.S. Patent No. 8,012,478), rheumatoid arthritis (U.S. Patent No. 7,786,274), etc. Therefore, antibodies of the invention, which are better (as compared to mAb 7E) anti-IL-20 antibodies, should also be effective in treating or preventing these IL-20 associated diseases or disorders.

Some embodiments of the invention relate to use of antibodies of the invention in treating or preventing IL-20 associated diseases or disorder, such as inflammatory diseases. In accordance with embodiments of the invention, a subject in need of treatment or prevention of an IL-20 associated disease is given a therapeutically effective amount of an antibody of the invention. The therapeutically effective amount is an amount sufficient to produce the desired clinical effects. One skilled in the art would appreciate that a pharmaceutically effective amount would depend on several factors, including patient's age, weight, condition, administration route, dosage form, and the antibody. However, finding an effective amount is routine practice that does not require inventive efforts, and one skilled in the art can find such effective amounts without undue experimentation.

Embodiments of the invention will be further illustrated with the following examples. These specific examples are for illustration only and are not intended to limit the scope of the invention. One skilled in the art would appreciate that various modifications and variations are possible without departing from the scope of the invention.

### EXAMPLES

### Selection of human V region framework sequences:

Human germ-line V_{L} and V_{H} sequences with the highest degree of homology with the mAb 7E framework regions were identified from the IMGT database (the International immunogenetics Information System®). The homology searches may be performed with BLAST or similar methods. The mAb 7E variable region sequences used as query sequences are available from the literature, such as U.S. Patent No. 7,786,274.

Human heavy chain framework sequences in the VH subgroup III (VH3) have been used in many humanized antibodies with success, and human light chain framework sequences of the VL κ subgroup II (Vκ2) are also shown to be good candidates. Therefore, the framework sequences of VH3 and Vκ2 subgroups were selected for the search for V_{H} and V_{L} frameworks, respectively. These searches identified IGHV3-72*01 and IGKV2D-29*02, respectively, as the VH and VL sequences most homologous to the corresponding heavy chain and light chain framework sequences in mAb 7E.

As shown in FIG. 1, the sequences of IGHV3-72*01 heavy chain framework regions differ from those in mAb 7E by 19 amino acids (the boxed residues), which corresponds to a 23.45% (19/ 81 total residues in the framework regions) variation. As shown in FIG. 2, the sequences of IGKV2D-29*02 light chain framework regions differ from those in mAb 7E by 10 amino acids (the boxed residues), which corresponds to a 13.16% (10/76 total residues in the framework regions) variation.

Even with these degrees of variations in the framework regions, an scFv (HH12) generated by grafting CDR sequences from mAb 7E into the IGHV3-72*01 and IGKV2D-29*02 sequences has a relatively good affinity for IL-20 (K_{D} = 2.68×10⁻⁹ M) (for comparison, mAb 7E, K_{D} = 7.81×10⁻¹⁰ M) (see Table 2 below). These results suggest that the framework regions can tolerate a relatively high degree of variations without impacting the CDR region conformations.

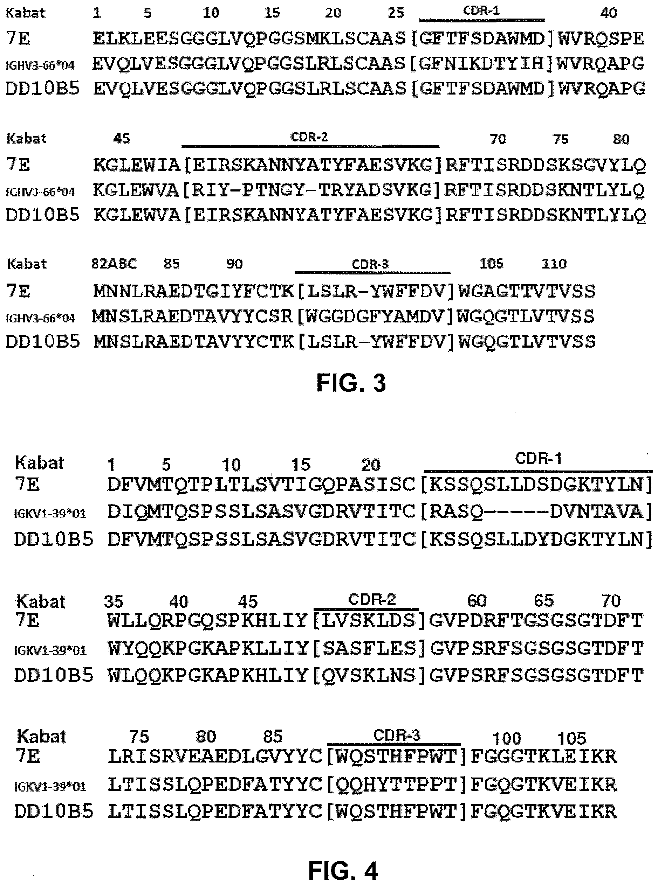

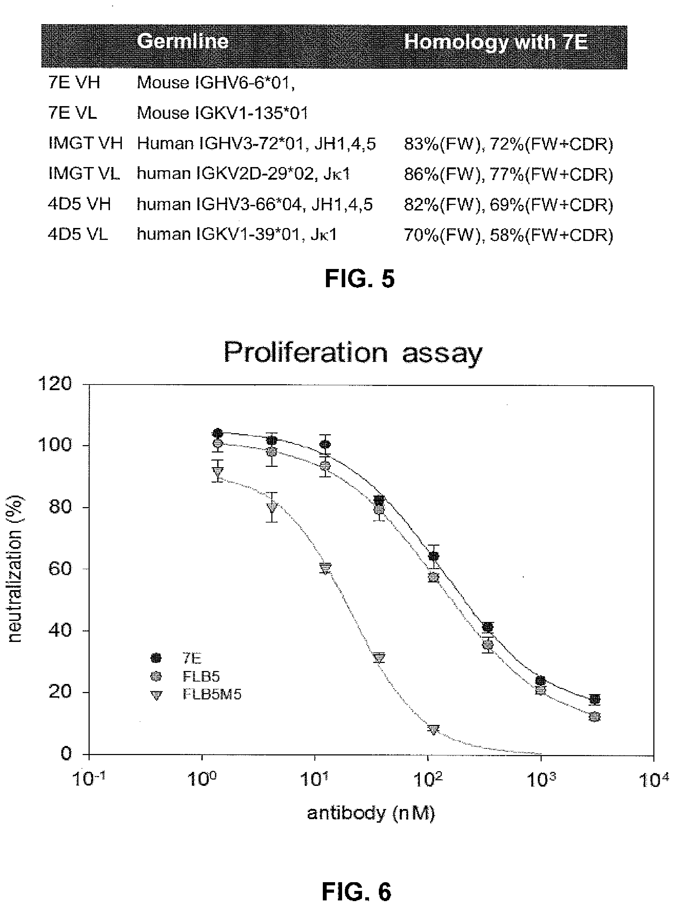

These two sequences, IGHV3-72*01 and IGKV2D-29*02, will be used as examples for the construction of humanized antibodies against Il-20. However, one skilled in the art would appreciate that other similar sequences with high degrees of homologies may also be used. Examples of other sequences, for example, may include human IGHV3-66*04 (Fig. 3) and human IGKV1-39*01 (FIG. 4). The homologies of these examples are shown in the table in FIG. 5.

One skilled in the art would appreciate that these specific examples are for illustration only, and are not intended to limit the scope of the invention. Using homology search methods and human immunoglobulin libraries to search for homologous sequences for a model antibody (in which the framework regions will be replaced with human sequences) involves only routine techniques and one skilled in the art would be able to identify suitable homologous sequences for the desired purposes. Any similar homologous human sequences may be used. Preferably, these human sequences have high homologies in frame work regions of the model antibody (e.g., 7E). In addition, variant sequences (i.e., "homologous variants") that are substantially homologous with a naturally occurring human heavy chain or light chain variable sequence may also be used. For example, the homologous variants may have 90% or higher (e.g., 93%, 95%, 98%, or 99%) identities with a human heavy chain or light chain variable sequence.

### HH12 scFv Construction and Display:

Based on the selected human heavy chain and light chain variable region homologs (e.g., IGHV3-72*01 and IGKV2D-29*02), an anti-IL-20 antibody or its fragments (e.g., scFv, Fab, etc.) may be constructed by grafting known CDR sequences from a known anti-IL-20 antibody (e.g., mAb 7E) into the homologous human heavy chain and light chain variable sequences. The following example uses CDR sequences from mAb 7E. However, one skilled in the art would appreciate that CDR sequences from any anti-IL-20 antibodies may be used.

In this particular example, an scFv fragment was generated. The scFv fragment, which is referred to as HH12, consists of complete human heavy-chain and light-chain framework sequences from IGHV3-72*01 and IGKV2D-29*02, respectively, and the six complete murine CDR sequences from mAb 7E. In this example, the HH12 scFv construct was assembled by overlap extension PCR. First, oligonucleotides having overlap sequences were prepared. Then, an equimolar mixture of the oligonucleotides (e.g., at a final concentration of 0.4 µM) was PCR-assembled using 0.5 µl of Pfx50 DNA polymerase and 5 µl of Pfx50 buffer (Invitrogen), according to the manufacturer's procedures.

After the assembly of the HH12 scFv construct using overlap extension PCR, a second PCR was performed using oligonucleotide primers to incorporate 5' SfiI and 3' NotI restriction sites. This produced a full-length HH12 scFv construct with the desired restriction sites at the 5' and 3' ends for directional subcloning into a modified phage display vector pCANTAB5e (Amersham Pharmacia Biotech). This vector may be used for the production of HH12 scFv.

### Back mutation:

For the expression of full-length antibodies in free style 293 cells, plasmid pTCAE8.3 was used for subcloning of the variable regions. This plasmid contains a DNA fragment encoding human κ C_{L} region and human γ C_{H} region. For mutations in the variable regions, individual oligonucleotides were synthesized to encode the desired mutations using PCR. These oligonucleotides include sufficient overlaps for PCR priming from the HH12 template. Next, the PCR products were gel-purified. Then, equimolar aliquots of the purified PCR products were combined for megaprime PCR to regenerate the full-length V_{L} (or V_{H}, if the mutation is in the heavy chain). The full-length V_{L} fragment was then subcloned into pTCAE8.3 vector for the expression of full-length antibodies containing the desired mutations.

### Look-through mutagenesis (LTM) Library Construction:

Look-through mutagenesis (LTM) is a multidimensional mutagenesis that allows one to simultaneously assess and optimize combinatorial mutations of selected amino acids in a target peptide segment. LTM has been successfully used to optimize CDR sequences. The mutagenesis process typically focuses on one or more CDR domains and explores the contributions, including synergistic contributions, of amino acid side chains in the antigen binding. *See*, Rajpal et al., "A general method for greatly improving the affinity of antibodies by using combinatorial libraries," P.N.A.S., 102(24), pp. 8466-8471, 2005.

To prepare the LTM library, individual oligonucleotides were synthesized to encode each amino acid substitution for each CDR position. Each oligonucleotide contains sufficient overlaps for PCR priming from the HH12 template. PCRs containing LTM oligonucleotide mixtures corresponding to individual CDRs were used to amplify LTM-substituted CDR fragments. Next, these PCR fragments were gel-purified, and equimolar aliquots of the purified fragments were combined for megaprime PCR to regenerate full-length variable regions. These full-length variable regions were inserted into the pCANTAB5e phagemid vector. The ligated DNA (the phagemid) was electroporated into *E. coli* TG1 cells to generate a library stock.

### Preparation of phage and selection of phage antibody libraries

For selection of phage (LTM) library with biopanning, the above-described library stock of E. coli was grown to the log phase. The phage particles displaying scFv were rescued by infection with M13KO7 helper phage (NEB) and amplified overnight in 2YTAK (2YT containing 100 µg/mL ampicillin and 25 µg/mL kanamycin) at 30°C. The phage was precipitated with PEG/NaCl (20% PEG 8000/2.5M NaCl), and then resuspended in PBS. The library was selected using biotinylated IL-20 and streptavidin-coated paramagnetic beads M280 (Dynal), as described below.

For selection of the LTM library, the biotinylated IL-20 at concentrations of 4.0×10⁻⁸ M, 1.0 ×10⁻⁹ M, 1.0 ×10⁻¹¹ M, 1.0 ×10⁻¹² M, and 1.0 ×10⁻¹³ M were used for selection rounds 1, 2, 3, 4, and 5, respectively. The mixture of the phages and the biotinylated IL-20 antigen was gently rotated for one hour at room temperature, and the phages bound to the biotinylatedIL-20 antigen were captured using 50∼100 µl of streptavidin-coated M280 magnetic beads for five minutes. After capture of the phages, the beads were washed a total of ten times (4×PBST (PBS containing 0.05% Tween 20), 2×PBSM (containing 2% skimmed milk powder), 4×PBS) using a Dynal magnetic particle concentrator. The third, fourth, and fifth washes were performed in competition with 1.4 µM IL-20 (non-biotinylated). Bound phages were eluted from the beads by sequential incubation with 1ml of 100mM triethylamine (TEA) for 30 minutes. Eluents were combined and neutralized with 0.5 ml of 1 M Tris HCl (pH 7.4) and half of the eluent was used to infect log phase *E. coli* TG1. The bindings, capture, wash, elution, and re-infection of E. coil were repeated for the desired number of cycles.

### Expression and Affinity Measurements of HH12 Variants

The genes encoding the V_{H} and V_{L} chains of HH12 and its mutants were inserted into the pTCAE8.3 expression vector to produce scFv constructs. Free style 293 cells (Invitrogen0 were transfected with the constructs. The vector was transfected into the host cells by lipofectamine 2000 in accordance with the attached instruction manual (manufactured by Invitrogen). After the full-length scFv was purified from the pooled supernatants, competition ELISA and BIACore assays were used to detect and assess epitope specificities and binding affinities.

### Combinatorial beneficial clone

From the above experiments, the mutant antibodies that can still bind to IL-20 would be identified, while mutants that lost binding affinities to IL-20 would likely not be identified. Analysis of the results reveals that three mutations in HH12 light chain CDR regions produced an antibody with an enhanced binding to IL-20. These three mutations include S32Y (in CDRL-1), L50Q (in CDRL-2), and D55N (in CDRL-2), which are shown as circled residues in FIG. 2.

These results also suggest that mutations at other residues in the CDR regions probably produced antibodies with relatively lower affinities such that they were not selected by phage panning, which tends to favor the tightest binder in the phage population. Therefore, it is likely that these other amino acids in the CDRs are relatively good fits for the binding interactions with IL-20 and no significant improvements can be realized by substitutions at these other locations.

In addition to the three "improved" substitutions in the CDR regions, the best antibody, FLB5M5, identified from the phage display-biopanning process also contains five beneficial mutations in the HH12 framework regions. These five amino acids are found to have been mutated from the residues in HH12 back to the corresponding residues in mAb 7E (i.e., back mutations). These five amino acids are: F2, V3, L36, K45, and H46, which are all in the light chain variable sequence, as shown in FIG. 2. The fact that back mutation produced better binders suggests that these residues in the framework regions contribute indirectly to the binding to IL-20 They probably contribute to the maintenance of proper conformations in the CDR regions.

The antibody, FLB5M5, identified from panning of the LTM library was found to bind tighter to IL-20 (as compared with mAb 7E), as shown in Table 2, and has a better therapeutic efficacy, as shown in FIG. 3. This finding (i.e., the humanized antibody is better than the natural antibody) is surprising because mAb 7E is a natural antibody produced by an immune system. Immune systems are known for their efficiency in producing "optimized" antibodies to bind with antigens. Therefore, humanization of an antibody is typically expected to produce a worse antibody in terms of binding with the target antigen.

The heavy chain variable region sequence (SEQ ID NO:4) and the light chain variable region sequence (SEQ ID NO:8) are shown in FIG. 1 and FIG. 2, respectively. An example of the entire heavy chain sequence (including the constant regions from human immunoglobulin) and the entire light chain sequence (including the constant region) are shown as SEQ ID NO: 9 and SEQ ID NO: 10, respectively, while the corresponding polynucleotide sequences that encode the SEQ ID NO:9 and SEQ ID NO:10 are shown as SEQ IS NO:11 and SEQ ID NO:12, respectively.

### Important residues in the CDR regions by alanine scanning

The above described phage panning of LTM library can identify optimized (i.e., tighter binders) antibodies. However, this phage panning approach gives only implication of the importance of certain amino acids in the CDR regions, as inferred from the lack of tighter binders when substitutions occurred at those locations. In order to positively assess the contributions of various amino acids side chains in the CDR regions to the binding of IL-20, alanine scanning was performed to replace individual amino acids in the CDR regions with alanine. If the original amino acid side chains are important for the binding, such alanine substitutions would reduce the binding affinities. Results from such an exemplary structure-activity relationship (SAR) study are shown in FIG. 5.

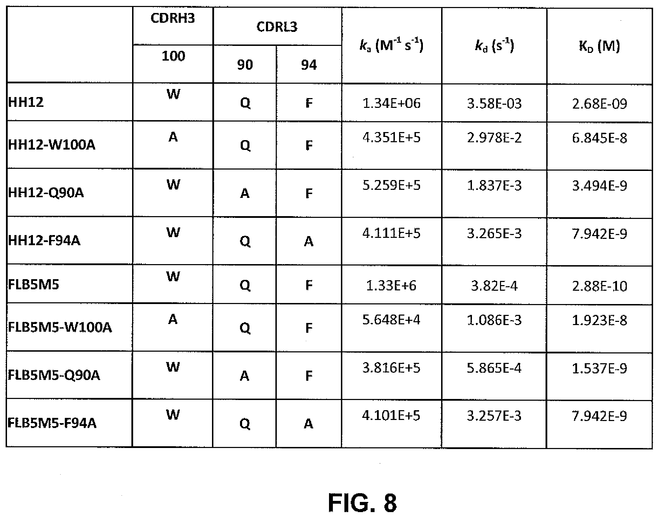

FIG. 8 shows the binding assay results of antibodies generated by alanine substitutions. Three residues in CDRH3 and CDRL3 of HH12 and FLB5M5 are found to be important. Specifically, W100 in CDRH3 is important because alanine substitution at this position substantially reduce the binding affinities of both antibodies (HH12 and FLB5M5). In CDRL3, F94 seems to be more important than Q90.

It was also found that alanine substitutions at other locations did not produce significant changes in the bindings to IL-20.

### IL-20 binding activity

The human IL-20-binding kinetics of each purified anti-IL-20 antibody was estimated by surface plasmon resonance measurements using the BIAcore T100 biosensor system. The anti-IL-20 Ab was captured on an anti-human IgG immobilized CM5 sensor chip. The immobilized level of anti-human IgG was about 9,000-10,000 RU and the capture level of anti-IL-20 antibody was about 350-400 RU. Binding was carried out at constant flow rates of 30µL/min of IL-20 at various dilutions in HEPES buffered saline (BIA certified) for 60 seconds. Dissociations were carried out by passing through HEPES buffer for 480 seconds. Regeneration of the surface was carried out by infecting 10 mM Glycine pH 2.0/1.5 (50:50) for 40 seconds.

The IL-20 affinity of each of the anti-IL-20 antibodies were calculated from an affinity binding curve fit using the predefined model (1:1 binding) provided by Biacore T100 evaluation software 2.0. The binding affinity data for mAb 7E, HH12, and FLB5M5 are summarized in Table 2. As can be seen from the data in Table 2, even though HH12 has a lower affinity to IL-20 than mAb 7E, FLB5M5 has a higher affinity (i.e., lower K_{D}) than mAb 7E.

**Table 2. Comparison of binding affinities to IL-20 of mAb 7E, HH12, and FLB5M5**

| **Specimen** | **kₐ (M⁻¹ s⁻¹)** | **k_{d} (s⁻¹)** | **K_{D} (M)** |
|---|---|---|---|
| 7E | 9.11E+5 | 7.11E-4 | 7.81E-10 |
| HH12 | 1.34E+6 | 3.58E-3 | 2.68E-9 |
| FLB5M5 | 1.33E+6 | 3.82E-4 | 2.88E-10 |

The fact that an artificial antibody, FLB5M5, binds several folds tighter to IL-20 than the binding between mAb 7E and IL-20 is truly unexpected. mAb 7E is a natural antibody generated by a mouse immune system. By cooperative actions of various cytokines and helper cells, the immune system is known for its efficiency in producing optimized antibodies to bind with antigens. Therefore, humanization of a natural antibody often produces "less-optimal" antibodies, i.e., humanized antibodies typically have lower affinities than the original antibodies (see e.g., U.S. Patent No. 8,597,647).

### IL20R2/IL22R BaF3 Proliferation assay

IL-20 is a pleitropic cytokine and has been shown to be involved in various inflammatory diseases and other disorders, and inhibition of IL-20 function has been shown to prevent or reduce various IL-20 associated diseases and disorder. Antibodies of the present invention bind IL-20 with very high affinities. Therefore, they should also be useful as therapeutics for the treatment and prevention of IL-20 associated diseases or disorders.

Some embodiments of the invention relate to methods for treating or preventing a disease associated with IL-20, which for example may include an inflammatory disease (e.g., rheumatoid arthritis), osteoporosis, cancer, stroke, and renal failure, estrogen deficiency (e.g., menopause), androgen deficiency (e.g., andropause), or cancer-induced osteolysis. A method in accordance with one embodiment of the invention may include administering to a subject in need of such treatment an effective amount of an antibody of the invention.

In this example, antibodies of the invention were tested for their abilities to inhibit or reduce IL-20 induced cell proliferation. Briefly, Ba/F3 cells stably transfected with full-length human IL-20 receptor complexes IL-22R and IL-20R2 to produce BaF-3(IL-20R2/IL22R) cells as the targets. Ba/F3 cells are murine precursor B cells of early lymphoblastoid cell lineage; they depend on IL-3 for viability and proliferation. These cells may be cultured in RPMI medium containing 10% fetal bovine serum and 1 ng/mL IL-3. Human recombinant IL-20 can induce the proliferation of these cells. This IL-20 induced cell proliferation system can be used to assess the abilities of the antibodies of the invention to inhibit the IL-20 functions, due to their bindings to IL-20.

In the proliferation assay, BaF-3(IL-20R2/IL22R) cells were seeded in the wells of microtiter plates at 10⁴ cells per well in the medium without IL-3 for 2 h at 37°C in a 5% CO₂ incubator. Then, BaF-3(IL-20R2/IL22R) cells were cultured with pre-incubated 300 pM human cytokine IL20 and an increasing amount of test antibodies (three-fold dilutions from 1000 to 0.15 nM) for another 72h. In this example, three antibodies were tested: mAb 7E, FLB5, and FLB5M5. FLB5 is an antibody that contains the three amino acid changes in the CDR regions shown in FIG. 2, as compared with HH12. However, FLB5 contains the same framework regions as those in HH12. In other words, FLB5 does not have the five "back mutated" amino acids in the framework regions, as compared with FLB5M5.

After the incubation, AlamarBlue (Promega), a colorimetric/fluorigenic growth indicator, was added to the cultures, and the cells were incubated for another 6 hrs. The microtiter plates were then read on a fluorometer with 530 nm excitation and 580 nm emission. The fluorescent readings were analyzed using SigmaPlot Software to find the half maximal response (EC₅₀) for the anti-human IL20 antibodies.

FIG. 6 shows results from the proliferation assay. As shown in FIG. 6, antibody FLB5 is as good as or slightly better than mAb 7E in inhibiting IL-20-induced proliferation, while FLB5M5 is significantly better than mAb 7E. The fact that a modified antibody has a tighter binding than a naturally produced antibody is unexpected.

The EC₅₀ values of FLB5M5 as compared with that of mAB 7E are shown in Table 3. These data show that FLB5M5 is several times better than mAb 7E in inhibiting IL-20-induced cell proliferation. Again, the fact that this artificially created antibody is more effective than the natural antibody mAb 7E is unexpected.

**Table 3. Comparison of neutralizing activity toward IL-20 of mAb 7E and FLB5M5**

| **Specimen** | **EC₅₀ (nM)** |
|---|---|
| 7E | 134.63 |
| FLB5M5 | 20.76 |

### Collagen-induced arthritis rat model:

As noted above, IL-20 is involved in imflammatory diseases and antibodies against IL-20 have been shown to be effective in reducing the inflammatory diseases such as theumatoid arthritis. Antibodies of the invention should also be useful in treating arthritis. This was test using a collagen-induced arthritis model in rats.

In this animal model, six-week-old male Sprague-Dawley (SD) rats were immunized with type II collagen on day 0 and day 7 to induce arthritis. For example, the rats were immunized with an emulsion containing equal parts of Freund's complete adjuvant, 4 mg/ml of heat-killed mycobacterium tuberculosis and bovine type II collagen solubilized at 2 mg/ml in 0.05 M acetic acid. Each rat was injected intradermally in its dorsum with 200 µl of the emulsion on day 0. On day 7, the rat received a booster dose subcutaneously in the base of its tail with 100 µl of the same emulsion. Rats injected with buffers without the collagen (blank) were as a negative control (no arthritis).

After the onset of arthritis, which typically occurs on day 10-13, the treatments were started. Rats were administrated twice per week for a total of three injections - i.e., animals of all treatment groups received a single bolus subcutaneous injection in the back on days 10, 14, and 18. The treatments were conducted at doses of 1 mg/kg (mpg), 3 mg/kg, and 9 mg/kg of antibody, hFLB5M5. Embrel (at 6 mg/kg) was used as a positive treatment control. The body weight (FIG. 4C), hind-paws thickness (PTH) (FIG. 4B and arthritic score (AS) (FIG. 4A) were assessed to evaluate the therapeutic efficacies at three doses of the antibody.

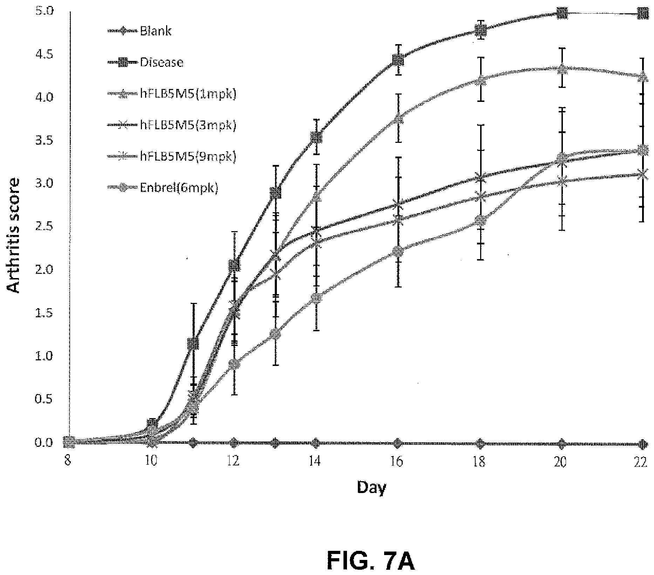

FIG. 7A shows results of the tests, as assessed with the arthritis scores (AS). As shown in FIG. 7A, administration of FLB5M5 produced substantial reductions in the AS, indicating that this antibody is effective in reducing the symptoms of arthritis. The FLB5M5 antibody is effective in reducing the AS even at 1 mg/kg, while at 3 mg/kg and 9 mg/kg, this antibody produced effects similar to the positive control (Embrel at 6 mg/kg). These results clearly show that FLB5M5 can be used to treat or prevent arthritis.

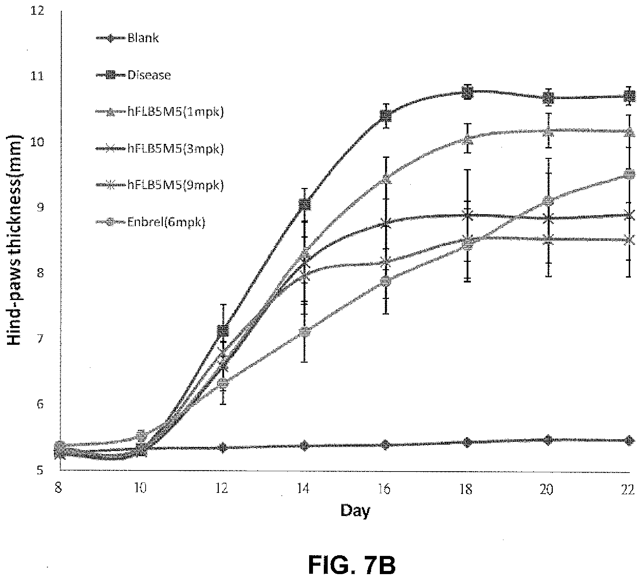

FIG. 7B shows results of hind-paw thickness assays. As shown in FIG. 7B, administration of FLB5M5 produced substantial reductions in hind-paw thickness, indicating that this antibody is effective in reducing the symptoms of arthritis. The FLB5M5 antibody is effective in reducing hind-paw thickness even at 1 mg/kg, while at 3 mg/kg and 9 mg/kg, this antibody produced effects similar to the positive control (Embrel at 6 mg/kg). These results clearly show that FLB5M5 can be used to treat or prevent arthritis.

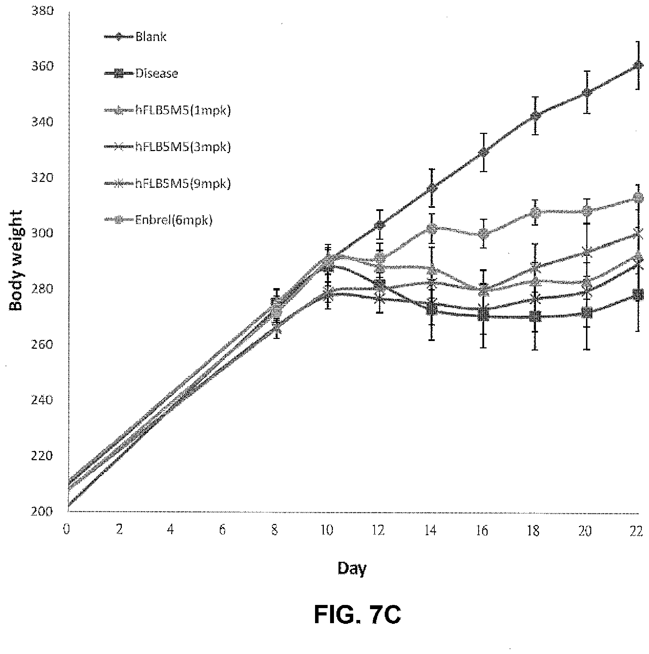

FIG. 7C shows results of body weight assays. As shown in FIG. 7C, administration of FLB5M5 produced substantial reductions in body weight, indicating that this antibody is effective in reducing the symptoms of arthritis. The FLB5M5 antibody is effective in reducing body weight even at 1 mg/kg, while at 3 mg/kg and 9 mg/kg, this antibody produced effects similar to the positive control (Embrel at 6 mg/kg). These results clearly show that FLB5M5 can be used to treat or prevent arthritis.

In addition to arthritis, antibodies against IL-20 are effective in the treatments of diseases associated with the IL-20 mediated signaling pathway including, but are not limited to osteoporosis, rheumatoid arthritis, cancer, stroke, or renal failure. In accordance with embodiments of the invention, the diseases may include osteoporosis, which can be caused by an inflammatory disease (e.g., rheumatoid arthritis), osteoporosis, cancer, stroke, and renal failure, estrogen deficiency (e.g., menopause), androgen deficiency (e.g., andropause), or cancer-induced osteolysis. That anti-IL-20 antibodies can be used to treat these diseases is known in the art, *see e.g.*, U.S. Patent No. 8,597,647, issued to Chang et al.

While the invention has been described with respect to a limited number of embodiments, those skilled in the art, having benefit of this disclosure, will appreciate that other embodiments can be devised which do not depart from the scope of the invention as disclosed herein. Accordingly, the scope of the invention should be limited only by the attached claims.

## Claims

1. A humanized antibody against IL-20, or a scFv, Fab, or F(ab')₂ fragment thereof, comprising:
a heavy chain variable region comprising SEQ ID NO:4; and
a light chain variable region comprising SEQ ID NO:8.

2. The humanized antibody against IL-20 according to claim 1, wherein the heavy chain in the humanized antibody comprises the sequence of SEQ ID NO:9, and the light chain in the humanized antibody comprises the sequence of SEQ ID NO:10.

3. A composition comprising the humanized antibody against IL-20, or a scFv, Fab, or F(ab')₂ fragment thereof, according to claim 1 or claim 2 for use in therapy.

4. The composition for use in therapy according to claim 3, wherein the therapy is against rheumatoid arthritis, osteoporosis, cancer, stroke or renal failure.

## Patentansprüche

1. Humanisierter Antikörper gegen IL-20 oder ein scFv-, Fab- oder F(ab')₂-Fragment davon, umfassend:
einen variablen Bereich der schweren Kette, der SEQ ID Nr. 4 umfasst; und
einen variablen Bereich der leichten Kette, der SEQ ID Nr. 8 umfasst.

2. Humanisierter Antikörper gegen IL-20 gemäß Anspruch 1, wobei die schwere Kette in dem humanisierten Antikörper die Sequenz von SEQ ID Nr. 9 umfasst und die leichte Kette in dem humanisierten Antikörper die Sequenz von SEQ ID Nr. 10 umfasst.

3. Zusammensetzung, die den humanisierten Antikörper gegen IL-20 oder ein scFv-, Fab- oder F(ab')₂-Fragment davon gemäß Anspruch 1 oder 2 umfasst, zur Verwendung in der Therapie.

4. Zusammensetzung zur Verwendung in der Therapie gemäß Anspruch 3, wobei die Therapie gegen rheumatoide Arthritis, Osteoporose, Krebs, Schlaganfall oder Nierenversagen gerichtet ist.

## Revendications

1. Anticorps humanisé contre IL-20, ou un de ses fragments scFv, Fab ou F(ab')₂, comprenant :
une région variable à chaîne lourde comprenant SEQ ID NO:4 ; et
une région variable à chaîne légère comprenant SEQ ID NO:8.

2. L'anticorps humanisé contre IL-20 selon la revendication 1, dans lequel la chaîne lourde de l'anticorps humanisé comprend la séquence de SEQ ID NO:9, et la chaîne légère dans l'anticorps humanisé comprend la séquence de SEQ ID NO:10.

3. Une composition comprenant l'anticorps humanisé contre l'IL-20, ou un fragment scFv, Fab, ou F(ab')₂ de celui-ci, selon la revendication 1 ou la revendication 2 pour une utilisation en thérapie.

4. La composition destinée à être utilisée en thérapie selon la revendication 3, dans laquelle la thérapie est contre l'arthrite rhumatoïde, l'ostéoporose, le cancer, l'accident vasculaire cérébral ou l'insuffisance rénale.
